**Europäisches Patentamt**

**European Patent Office**

**Office européen des brevets**

(11) Publication number : **0 297 038 B1**

(12) **EUROPEAN PATENT SPECIFICATION**

(45) Date of publication of patent specification :
**20.05.92 Bulletin 92/21**

(51) Int. Cl.$^5$ : **A61K 47/02, A61K 47/44**

(21) Application number : **88810396.7**

(22) Date of filing : **10.06.88**

(54) Topical oil compositions of increased viscosity and method of preparing same.

(30) Priority : **16.06.87 US 62939**

(43) Date of publication of application :
**28.12.88 Bulletin 88/52**

(45) Publication of the grant of the patent :
**20.05.92 Bulletin 92/21**

(84) Designated Contracting States :
**AT BE CH DE ES FR GB GR IT LI LU NL SE**

(56) References cited :
**EP-A- 0 141 410**
**DE-A- 2 262 139**
**FR-A- 2 076 990**
**US-A- 3 525 689**

(56) References cited :
**US-A- 4 284 630**
**Die Pharmazie, Vol. 22 (1967), p. 44-46**
**J. Soc. Cosmet. Chem ; Vol. 21 (1970) pages 347, 348**

(73) Proprietor : **WARNER-LAMBERT COMPANY**
**201 Tabor Road**
**Morris Plains New Jersey 07950 (US)**

(72) Inventor : **Geria, Navin Manohar**
**32, Mountainview Road**
**Warren New Jersey 07060 (US)**

(74) Representative : **Jones, Michael Raymond**
**HASELTINE LAKE & CO. Hazlitt House 28**
**Southampton Buildings Chancery Lane**
**London WC2A 1AT (GB)**

## Description

This invention relates to novel animal, vegetable and mineral oil compositions having increased viscosity formulations containing them and a method of making them. These compositions comprise a major proportion of oil and an inorganic complexing agent capable of effectuating a viscosity increase without substantially effecting other properties of the oil.

Oil compositions of increased viscosity are used as a base component or starting material for the formation of a number of products. Such oil based products are useful in the food, pharmaceutical and cosmetic industries. In general, it is desirable to thicken or increase the viscosity of oils to facilitate the formation of suspensions, lotions, creams, greases, salves, ointments and butter or margarine like products.

The complexed oils of the present invention can be used as a base to form topical pharmaceutical products. The oils themselves have therapeutic value particularly in the treatment of dry skin. Topical products such as lotions, creams and ointments are utilized to deliver controlled levels of moisture to rehydrate the skin. The oil portion then acts to seal in moisture thereby preserving a moisturized effect.

Topical products are also utilized to deliver therapeutic agents directly to the skin surface. Such products are used to treat muscular pain, local infection, dermititus, nappy (diaper) rash and similar conditions. The smoothing, coating penetrating action of the oils, oil in water and water in oil emulsions is combined with the beneficial effect of the therapeutic agent.

Well known techniques utilized to increase oil viscosity include: emulsifying oils with an aqueous component and thickening agent; combining oil with a polymeric material or a higher melting wax having a long chain molecular structure; and hydrogenation of unsaturated oils to increase viscosity through molecular interaction. Each of these techniques has its own drawbacks. For example, emulsions are formed with the addition of emulsifying agents such as fatty alcohols and hydrocolloids which may be detrimental to other desired ingredients such as therapeutic agents. Addition of polymeric materials and waxes to oil generally requires high heat for a prolonged period which may degrade the oil or other components of the final product. Hydrogenation is expensive, produces saturated fats and changes the character of the oil.

From US-A-4 284 630 there are known stable water-in-oil emulsions in which magnesium oxide or magnesium hydroxide is added to the aqueous phase as a stabllising agent.

From Hager's Handbuch der Pharmazeutischen Praxis, 4th Edition, pages 280 and 281, there is known the use of Betonite, e.g. Veegum®, in the aqueous phase of oil-in-water emulsions as a viscosity increasing agent.

From Pharmazie, Vol. 22(1967) pages 44-46 there is known that the viscosity of sunflower oil is increased when the commercially available colloidal silica dioxide Aerosil® is admixed with it.

According to the present invention there is provided a composition which comprises:-

an oil and an inorganic complexing agent selected from magnesium trisilicate, calcium carbonate, calcium silicate, a co-dried gel of aluminium hydroxide and magnesium carbonate, magnesium hydroxide, magnesium carbonate, aluminium hydroxide, ground limestone or ground oyster shells or mixtures thereof, which is in particulate form, is insoluble in oil and which is associated with the oil such that the viscosity of the oil is increased; and

water,

which oil/agent association and water are in the form of an emulsion.

It has been unexpectedly discovered that the addition of certain inorganic compounds to oil will produce a gel-like structure or complex within the oil and that the oil complex will form formulations for hydrous for lotions, and creams. The oil complex providing a stable viscosity increased base for , for example, hydrous topical delivery systems. The viscosity of such formulations, eg. topical products, is controlled by the amount of oil complex used in the product and by the viscosity of the oil complex which is proportional to the concentration of inorganic compound.

In particular, it has been found that a composition having increased viscosity is produced from an oil, an inorganic complexing agent, and water wherein the oil and the complexing agent are combined to form a complex of increased viscosity and water blended into the mixture to form an emulsion. The increased viscosity of the oil complex provides for the formulation of formulations, e.g. lotions and creams, without the use of thickeners. In particular, the compositions of the present invention may be used to provide formulations such as a topical pharmaceutical formulation which comprises: an oil; an inorganic complexing agent as defined which is insoluble in the oil; wherein the oil and complexing agent are combined to form a complex of increased viscosity and then blended with water to form an emulsion.

Throughout the specification and claims the ensuing terms are defined as follows:

"Marine oils" means oils from fresh and salt water marine fish and animals and includes : fish liver oils, cod liver oil, fish oil, whale oil, seal oil and oils containing omega-3 fatty acids.

"Therapeutic" means of or relating to the treatment of disease or disorders.

Throughout the specification and claims the terms inorganic complexing agent and inorganic complexing compound shall be used interchangeably to mean an inorganic compound which forms a complex or is otherwise associated with the oil to produce a gel-like structure with oil.

While the invention is not to be limited to theoretical considerations, it is believed that the incorporation of the inorganic complexing agent into the oil provides a particle surface which interacts with the oil molecules in such a way that one portion of the oil molecule is attracted to and becomes associated or complexed with the particle surface while the remainder of the oil molecule extends away from the particle. This phenomenon produces what is termed a "solvated particle". The ends of the oil molecules that extend away from the solvated particle are available to interact with remaining oil or with other solvated particles of complexing agent. These interactions cause the oil and complexing agent mixture to develop a weak gel-like structure, thereby forming an oil composition having increased viscosity. The structure is believed analogous to that of a micelle in which the solid particle of inorganic complexing agent is held inside a pseudo-micelle. The structure formed provides a viscosity increased oil complex suitable for forming a therapeutic topical composition of increased viscosity.

The oils useful in the present invention are varied and may be of animal, vegetable or mineral origin. Methods of producing oils are known and not a subject of the present invention. Animal oils are derived from the organs and tissues of animals and may be collected through extraction, heating and/or expressing processes. Vegetable oils are usually derived from the seeds of various plants and are generally produced by extraction or pressing processes. Mineral oils are derived from petroleum and are recovered through various refining processes. Throughout the specification and claims, the term "oil" shall be defined as any oil of animal, vegetable or mineral origin in liquid form at the time of addition of the inorganic complexing agent.

The oils useful in the present invention may be food grade edible oils or nonedible oils. For example, food grade oils would be particularly useful in edible pharmaceuticals and food products. Nonedible and edible oils would be useful in topical pharmaceuticals, cosmetics, personal care products and in lubricants.

Illustrative examples of oils useful in the present invention include animal oils such as the marine oils: fish oil, whale oil, fish liver oil, seal oil, cod liver oils and oils containing at least one omega-3 fatty acid; vegetable oils such as castor oil, linseed oil, sunflower oil, soybean oil, olive oil, peanut oil, rapeseed oil, corn oil, safflower seed oil, cottonseed oil, coconut oil, palm oil, palm kernel oil and oils containing at least one omega-3 fatty acid; mineral oils such as white mineral oil. Any of the oils may be used individually or in mixtures.

The complexing agent is characterized by being insoluble in oil, being inorganic and by its ability to form a complex with the oil such that the viscosity of the oil is increased.

The inorganic complexing agents must be incorported into the oil in particulate form. The particle size may vary widely but must be of adequate size to enable incorporation into the oil without exhibiting a gritty or sandy feel. In addition, the particle size must be adequate to enable complex formation with the oil. Exemplary average particle size ranges may be from 0.1 micrometres to 300 micrometres, preferably 0.5 micrometres to 150 micrometres and most preferably 0.5 micrometres to 100 micrometres.

The inorganic complexing agent are selected from a wide range of compounds that are insoluble in oil and provide complexation of oil when admixed with oil. The inorganic complexing agents are selected from magnesium trisilicate, magnesium hydroxide, calcium carbonate, calcium silicate, a co-dried gel of aluminum hydroxide and magnesium carbonate, magnesium carbonate, aluminium hydroxide, ground limestone, ground oyster shells, and mixtures thereof.

The change in oil viscosity with increasing complexing agent concentration is a continuum. Free flowing liquids are formed at low complexing agent concentrations and thick, essentially nonflowing compositions having a texture like that of petroleum jelly are formed at higher complexing agent concentrations. The "gel-like" structure of the present invention has very weak elastic properties and will readily deform when a slight force is applied.

The ratio of inorganic complexing agent to oil will determine the viscosity or degree of thickening of the oil. A complexing agent to oil ratio of 1:99 to 1:10 will produce liquids having a gradual increase in viscosity. Complexing agent to oil ratios of 1:9 to 1:3 will produce liquids with the consistency of syrups. Complexing agent to oil ratios of 1:2.5 to 1:1 will produce semisolids having the consistency of creams to ointments. Complexing agent to oil ratios greater than 1:1 will produce solids. The texture of the solids will become granular and change to a powder as the complexing agent to oil ratio increases beyond 1.2:1. In general, complexing agent to oil ratios of 1:99 to 1.2:1 will produce use for compositions.

The oil complex is present in an amount of 5% to 90%, preferably 10% to 90% and most preferably 10% to 80% by weight of the total composition.

Water is used in the present invention to form an emulsion with the complexed oil and also acts as a skin hydrating agent for topical formulations. Water is present in an amount of 10% to 95%, preferably 10% to 85% and most preferably 15% to 80% by weight of the composition.

There may also be incorporated in the topical formulations of the present invention additional nonessential ingredients such as colourants, humectants, therapeutic agents, fragances, lubricants, preservatives, emulsifiers, surfactants and neutralizing agents. The foregoing list of materials is illustrative, other ingredients may be included herein.

Therapeutic agnets when used in the formulations of the present invention may be selected from a wide variety of medications and their acid addition salts. Both organic and inorganic salts may be used. Exemplary acid salts include hydrochloride, hydrobomide, benzoate, maleate, tartrate, acetate, succinate, citrate and sulphate. Suitable categories of medications that may be employed in the formulations may vary widely and generally represent any stable medication suitable for topical application. The therapeutic agents may be used alone or in mixtures. Illustrative categories and specific examples include but are not limited to:

Analgesics;-camphor, menthol, methyl salicylate, methyl nicotinate, eucalyptus oil, cyclomethycaine sulphate, eugenol, oleoresin capsicum, turpentine oil, pine oil and trolamine salicylate;

Anesthetics;-benzocaine, lidocaine, tetracaine, tripelennamine and dibucaine;

Antibacterials and Antiseptics;-chloroxylenol, 8-hydroxyquinoline sulphate, chlorothymol, cresol salicylic acid, benzoyl peroxide, silver sulphadiazine, erythromycin, meclocycine sulphosalicylate, tretinoin, tetracycline, choromycetin, becitracin, bacitracin zinc, neomycin, neomycin sulphate, nitrofurazone, garamycin, polymixin B sulphate, phenol, mafenide acetate, sulphacetamide, chloroxylenol, benzyl alcohol, diclonine, dimethisoquin, butamben, diperodon, chlortetracycline hydrochloride, gramicidin, oxytetracycline, hexylresorcinol and resorcinol;

Antifungals;-tolnaftate, undecylenic acid, zinc undecylenate, calcium undecylenate, caprylic acid, sodium propionate, sulphur, benzoic acid, boric acid, zinc stearate, zinc oxide, clioquinol, iodoquinol, iodochlorhydroxyquin, nystatin, clotrimazole, haloprogin, econazole nitrate, miconazole nitrate, amphotericin B, ciclopirox olamine and triacetin;

Antihistamines;-diphenhydramine, tripelennamine, pyrilamine, phenyltoloxamine, chlorpheniramine and methapyrilene;

Steroids and Hormones;-hydrocortisone, triamcinolone acetonide, hydrocortisone acetate, betamethasone valerate, betamethasone 17-benzoate, diflorasone diacetate, fluocinolone acetonide and fluorouracil;

Skin protectants;-allantoin, cocoa butter, dimethicone, glycerin, shark liver oil, zinc oxide, zinc carbonate, zinc acetate and calamine;

Sun screen agents;-aminobenzoic acid, cinoxate, diethanlamine, p-methoxcinnamate, digalloyl trioleate, dioxybenzone, ethyl 4-[bis(hydroxypropyl)] aminobenzoate, 2-ethylhexyl 2-cyano-3,3-diphenylacrylate, ethylhexyl p-methoxycinnamate, 2-ethylhexyl salicyate, glyceryl p-aminobenzoate, homosalate, lawsone with dihydroxyacetone, menthyl anthranilate, oxybenzone, padimate A, padimate O, 2-phenylbenzimïdazole-5-sulfphonic acid, red petrolatum, sulisobenzone, titanium dioxide and triethanolamine salicylate.

The weight percent of the medication based on the weight of the formulation, is 0.1% to 25%, preferably 0.1% to 20% and most preferably 0.1% to 15% which amounts will vary depending on the therapeutic dosage permitted.

Preservatives such as benzoic acid, sorbic acid methylparaben, propylparaben, dl-alpha tocopherol, butylated hydroxy toluene (BHT), and ethylene-diaminetetracetic acid (EDTA). Preservatives are generally present in amounts up to 1% and preferably from 0.01% to 0.5% by weight of the formulation.

Colourants useful in the formulations include the pigments which may be incorporated in amounts of up to 2% by weight of the formulation. A preferred pigment, titanium dioxide, may be incorporated in amounts of up to 1% by weight. Also, the colourants may include other dyes suitable for food, drug and cosmetic applications, and known as F.D. & C. dyes. The materials acceptable for the foregoing spectrum of use are preferably oil soluble. A full recitation of all F.D. & C. and D. & C. colourants and their corresponding chemical structures may be found in the Kirk-Othmer Encyclopedia of Chemical Technology, 3rd edition, in Volume 6, at pages 561-595.

Neutralizing agents useful in the formulations include phosphoric acid, triethanol amine, citric acid, sodium citrate, sodium phosphate, acetic acid, sodium acetate and mixtures thereof. Neutralizing agents may be present in amounts of 0.01% to 0.5% by weight of the formulation.

A variety of materials may be utilized as emulsifiers, including high molecular weight polyethylene glycols, fatty alcohols such as stearyl alcohol and myristyl alcohol. These materials are generally present in amounts of 0.1% to 15% by weight of the formulation and preferably in amounts of 1% to 10%.

Suitable hymectants may be any of those well known in the art. Examples of useful humectants include glycerin, propylene glycol, polyethylene glycol, polyhydric alcohols and mixtures thereol, to name a few. Preferably, glycerin is used. These materials may be incorporated in the formulations in amounts of 0.1% to 30% by weight of the formulation and preferably in amounts of 3% to 20%.

Numerous surfactants, and preferably non-ionic surfactants, may be added for their intended purpose. Among those preferred are polyalkanolamines such as triethanolamine, polyethylene glycol stearate, polyethylene glycol laurate, polyoxyethylene and polyoxypropylene compounds, e.g. as derivatives of sorbitan and fatty alcohol esters, fatty acid esters of polyhydric. alcohols and amine oxides; anionic surfactants, such as alkyl carboxylates, acyl lactylates, sulphuric acid esters (e.g. sodium lauryl sulphate), 5 ester-lined sulphonates, and phosphated ethyxylated alcohols; cationic surfactants, such as monoalkyl and dialkyl quaternary ammonium salts, amidoamides and aminimides. These various surfactants, when compatible, can be added as mixtures to the formulations and are generally present in amounts of 0.1% to 15% by weight of the formulation.

Lubricating agents may be used when desired in the formulations. They include silicone oils or fluids such as substituted and unsubstituted polysiloxanes, e.g. dimethyl polysiloxane, also known as dimethicone, cyclomethacone, fatty acids ($C_7$ to $C_{24}$) and fatty acid esters such as stearic or palmitic. Stearic acid is particularly useful when the formulation is to be used as a topical preparation. The lubricating agents, when incorporated in a topical composition, are generally present in amounts of 0.1% to 30% by weight of the formulation and preferably in amounts of 1% to 10%.

Preservatives such as alkyl and aryl parabens and subtituted phenols are also useful additives. Examples of the preferred parabens are the methyl, propyl and butyl parabens useful in ranges of 0.1 to 0.25%. In a preferred embodiment, a combination of methyl, propyl and butyl paraben may be used in the respective ranges of 0.1% to 0.25%, 0.02% to 0.2% and 0 to 0.05%. Examples of the useful substituted phenols include chlorosubstituted phenoxy phenols, such as 5-chloro-2(2,4-dichlorophenoxy)phenol, hexachlorophene, triclosan and dichlorophene, among others.

Other useful preservatives include mercury derivatives, such as phenylmercuric acetate; quarternaries, such as benzethonium chloride, benzalkonium chloride and cetyl triethyl ammonium bromide; acids, such as sorbic acid; and a variety of other preservatives such as Kathon CG, a trademark of Rohm & Haas Co. which comprises a mixture of 5-chloro-2-methyl-4-isothiazolin-3-one and 2-methyl-4-isothiazlin-3-one.

Other conventional additives may be utilized, such as fragrances, and other additives.

The compositions of the present invention are prepared by admixing the inorganic complexing agent with the oil until a uniform homogeneous mixture is obtained and admixing water with the mixture to form a uniform emulsion.

The inorganic complexing agent interacts with the oil to increase viscosity and form a gel-like structure.

The admixing of oil and inorganic complexing agent may be at low shear or high shear. Low shear mixing is preferred as it is less likely to cause degradation of oil components. The mixture of oil and inorganic complexing agent may be at a temperature just above the freezing point of the oil up to just below the decomposition temperature of the oil. In general mixing of the oil and inorganic complexing agent occurs at 15°C to 70°C, preferably from 15°C to 50°C and most preferably from 15°C to 30°C. Water is then admixed with the oil complex until a uniform emulsion or hetergeneous mixture is formed. In general mixing of the oil complex and water occurs at 15°C to 75°C, eg 15 to 70°C, preferably from 15°C to 50°C and most preferably from 15°C to 30°C.

Optional ingredients when present may be admixed with the oil complex, the water or the oil complex and water emulsion.

The mixing of the oil and inorganic complexing agent to form a complex and the further mixing with water may be performed under an inert atmosphere such as nitrogen or carbon dioxide. The preparation of compositions containing oxidizable materials such as omega-3 acids and oxidizable therapeutic agents is preferably conducted in an inert atmosphere.

In general, the process for increasing the viscosity of oils comprises, contacting the oil with an inorganic complexing agent, said complexing agent forming a viscosity increasing complex with the oil.

The oil compositions of increased viscosity may be stored for future use or formulated with conventional additives to prepare medicated and nonmedicated formulations which offer a variety of textures from a liquid to a grease, cream, lotion, ointment or other forms to suit particular applications. The structure formed between the oil and inorganic complexing agent is stable in the presence of water permitting complexed oil and water emulsions to be formed. The novel compositions of this invention may be utilized in pharmaceuticals, cosmetics, personal care products, foods, agriculture, lubricants and solvents.

The present invention is further illustrated by the following examples. All parts and percentages in the examples and throughout the specification and claims are by total weight of the composition unless otherwise indicated.

In the following examples 1 and 2, the viscosities were measured on a Brookfield model LV-1 viscometer. These measurements represent relative and not "absolute" or "true" measures of viscosity. Viscosity measurements taken with the Brookfield using different spindles will vary. Example 2, Run 11 demonstrates that viscosities measured on spindle 2 will be, on the average, approximately 2.75 times higher than viscosities measured on spindle 3 for the same mixtures. The viscosities reported in the following examples must be interpreted in view of the above information.

EXAMPLE 1

(Inventive Runs 1 to 8 and Controls)

This example demonstrates the effect of magnesium trisilicate (MgT) content on the viscosity of mineral oil (MO) and fish oil (FO) in Pa.s as measured on a Brookfield model LV-1 viscometer.

The compositions of Table 1 were prepared by admixing magnesium trisilicate in the specified ratios with the particular oil until a uniform mixture was obtained.

Table 1
Viscosity of Magnesium Trisilicate
Fish and Mineral Oil Suspensions

| Run | Ratio MgT/MO | Spindle # | Viscosity $10^{-3}$ Pa.s at Given RPM (Evolutions per minute) | | | |
|---|---|---|---|---|---|---|
| | | | 6 | 12 | 30 | 60 |
| 1 | 5/95 | 1 | 70 | 72.5 | 70 | 56 |
| 2 | 10/90 | 2 | 300 | 225 | 160 | 125 |
| 3 | 20/80 | 3 | 6,140 | 3,900 | 1,940 | 1,180 |
| 4 | 30/70 | 4 | -- | 22,000 | 16,400 | 9,300 |
| | Ratio MgT/FO | | | | | |
| 5 | 5/95 | 1 | 75 | 75 | 58 | 52 |
| 6 | 10/90 | 1 | 292 | 200 | 128 | 98 |
| 7 | 20/80 | 3 | 3,700 | 2,150 | 1,172 | 680 |
| 8 | 30/70 | 4 | 32,000 | 17,000 | 8,200 | 4,600 |

Controls

| | | | | | | |
|---|---|---|---|---|---|---|
| Fish Oil | | 1 | 35 | 30 | 28 | 30 |
| Mineral Oil | | 1 | 30 | 22.5 | 22 | 22 |

In both the fish oil and mineral oil, a 5:95 ratio of MgT to oil effectively doubles the viscosity of the uncomplexed oil. In Runs 1 to 8, as the concentration of MgT increases the viscosity of the mixtures increase dramatically. The suspensions formed in Runs 1 to 8 are shear thinning. Shear thinning results in a decrease in viscosity as the shear, in this case RPM of the spindle, is increased. This behavior is typical of suspensions which have structure. As the shear is increased, the structure is broken down thus giving a lower viscosity.

Runs 1 to 8 all demonstrate the presence of a gel-like structure and increased viscosity.

EXAMPLE 2

(Inventive Runs 9 to 14)

This example demonstrates the effect of inorganic complexing (IC) agents on the viscosity of mineral oil (MO) and fish oil (FO) as measured on a Brookfield model LV-1 viscometer.

The compositions of Table 2 were prepared by admixing the indicated inorganic complexing agent in the specified ratios with either fish oil or mineral oil until a uniform mixture was obtained.

Table 2

Viscosity of Fish and Mineral Oil Suspensions

| Run | Materials | Ratio IC/Oil | Spindle | Viscosity $10^{-3}$ Pa.s at Given RPM | | | |
|-----|-----------|--------------|---------|-------|-------|-------|-------|
| | | | | 6 | 12 | 30 | 60 |
| 9 | (1)/FO | 20/80 | 3 | 400 | 400 | 200 | 180 |
| 10 | (1)/MO | 20/80 | 2 | 1,000 | 700 | 430 | 295 |
| 11 | $CaCO_3$/MO | 20/80 | 2 | 3,700 | 2,175 | (2) | (2) |
| | | | 3 | 1,300 | 825 | 440 | 420 |
| 12 | $Mg(OH)_2$/FO | 20/80 | 3 | 100 | 100 | 60 | 70 |
| 13 | $Mg(OH)_2$/MO | 20/80 | 2 | 150 | 150 | 120 | 90 |
| 14 | $CaCO_3$/FO | 20/80 | 2 | 150 | 125 | 100 | 80 |

Controls

| | | | | | | | |
|-----|-----------|--------------|---------|-------|-------|-------|-------|
| Fish Oil (FO) | | | 1 | 35 | 30 | 28 | 30 |
| Mineral Oil (MO) | | | 1 | 30 | 22.5 | 22 | 22 |

(1) = Aluminum hydroxide/calcium carbonate co-dried gel

(2) = Beyond range of instrument using spindle 2

Aluminium hydroxide/calcium carbonate co-dried gel shows a moderate effect on the viscosity of both mineral oil and fish oil. Calcium carbonate demonstrates a strong effect on the viscosity of mineral oil and a weaker, moderate effect on the viscosity of fish oil. Magnesium hydroxide demonstrates a weak moderate effect on the viscosity of both fish oil and mineral oil. All of the runs demonstrate shear thinning. Shear thinning is a drop in viscosity as shear, in this case RPM, is increased. The increases in viscosity of the inventive runs are caused by the structure developed around and between suspended particles. Calcium carbonate forms a strong structure containing composition with mineral oil but a weaker structure containing composition with fish oil.

EXAMPLE 3

(Inventive Runs 15 to 20)

This example demonstrates the effect of the inorganic complexing agent magnesium trisilicate (MgT) on various oils.

The compositions of Table 3 were prepared by admixing magnesium trisilicate in the specified ratios with the particular oil until a uniform mixture was obtained.

## Table 3

### Mixtures of Magnesium Trisilicate and Various Oils

| Run | Oil | Ratio MgT/Oil | Product Consistency |
|---|---|---|---|
| 15 | Peanut | 40:60 | Semisolid - salve |
| 16 | Safflower | 40:60 | Semisolid - salve |
| 17 | Corn | 40:60 | Semisolid - salve |
| 18 | Olive | 40:60 | Semisolid - salve |
| 19 | Castor | 20:80 | Viscous paste |
| 20 | Cod liver | 20:80 | Viscous paste |

All of the oils tested form a structure with magnesium trisilicate resulting in highly viscous compositions.

EXAMPLE 4

(Comparative Runs A to B)

This example demonstrates the effect of inorganic compounds which do not complex with oils.

The compositions of Table 4 were prepared by admixing various noncomplexing inorganic compounds (NIC) in the specified ratios with fish oil (FO) until a uniform mixture was obtained.

## Table 4

### Mixtures of Inorganic Compounds with Fish Oil

| Run | Comparative Inorganic Compound | Ratio NIC:FO | Product Consistency |
|---|---|---|---|
| A | Veegum ® (1) | 20:80 | Particulates settle. No significant viscosity increase |
| B | Colloidal silica | 20:80 | Particulates settle. No significant viscosity increase |

(1)Veegum ® = complex magnesium aluminum silicate a standard article of commerce sold as Veegum by the R.T. Vanderbilt Co, Inc., New York, New York.

None of the inorganic compounds tested above formed complexes with fish oil. The compounds settled to the bottom of the solution and did not cause a significant increase in oil viscosity.

EXAMPLE 5

(Inventive Run 21)

This example demonstrates a method for preparing a topical moisturizing lotion. The ingredients are mixed in the order indicated

| No. | Ingredient | | Percent (w/w) |
|-----|-----------|---|---------------|
| 1. | Fish oil complex | | 12.00 |
| | Fish oil | 60% w/w | |
| | Magnesium trisilicate 40% w/w | | |
| 2. | Lubricant | | 0.30 |
| 3. | Neutralizing agent | | 0.03 |
| 4. | Fragrance | | 0.05 |
| 5. | Preservative | | 0.20 |
| 6. | Water (to 100%) | | 87.42 |
| | Total | | 100.00 |

The fish oil complex is formed by mixing the fish oil and magnesium trisilicate until a homogeneous mixture of increased viscosity is obtained. The emulsifying agent is added to the oil complex heated to 75°C and mixed until uniform. The neutralizing agent and preservative are combined with water heated to 75°C. The water and oil composition are stirred together to homogenize. The lotion is cooled to 40°C and fragrance added with mixing. The final lotion has a smooth feel and moisturizing effect.

EXAMPLE 6

(Inventive Run 22)

This example demonstrates a method for preparing a topical moisturizing cream. The ingredients are mixed in the order indicated.

| No. | Ingredient | | Percent (w/w) |
|-----|-----------|---|---------------|
| 1. | Fish oil complex | | 70.00 |
| | Fish oil | 60% w/w | |
| | Magnesium trisilicate 40% w/w | | |
| 2. | Surfactant | | 2.00 |
| 3 | Fragrance | | 0.50 |
| 4 | Preservative | | 0.32 |
| 5 | Purified water (to 100%) | | 27.18 |
| | Total | | 100.00 |

The fish oil complex is formed by mixing the fish oil and magnesium trisilicate until a homogeneous mixture of increased viscosity is obtained. The oil complex is heated to 70°C and admixed with the preservative and surfactant until a homogenized mixture is obtained and then water is heated to 70°C and admixed with the oil composition. The both is cooled to 40°C and fragrance added with mixing.

EXAMPLE 7

A sum screen lotion according to this invention was obtained from the components below blended together as indicated.

| | | Percent (w/w) |
|-----|-----------|---------------|
| 1. | Moisturizing Lotion from Example 5 | 90 |
| 2. | Aminobenzoic Acid | 10 |

9

EXAMPLE 8

Anesthetic Cream Formulation

An anesthetic cream formulation according to this invention was obtained from the components below blended together as indicated.

|  | | | Percent (w/w) |
|---|---|---|---|
| 1. | Moisturizing Cream from Example 6 | | 98.00 |
| 2. | Benzocaine | | 2.00 |

## Claims

### Claims for the following Contracting States : AT, BE, FR, DE, IT, LU, NL, SE, CH AND GB

1. A composition which comprises:-
an oil and an inorganic complexing agent selected from magnesium trisilicate, calcium carbonate, calcium silicate, a co-dried gel of aluminium hydroxide and magnesium carbonate, magnesium hydroxide, magnesium carbonate, aluminium hydroxide, ground limestone or ground oyster shells or mixtures thereof, which is in particulate form, is insoluble in the oil and is associated with the oil such that the viscosity of the oil is increased; and
water
which oil/agent association and water are in the form of an emulsion.

2. A composition according to claim 1, wherein the inorganic complexing agent is magnesium trisilicate.

3. A composition according to claim 1 and 2, wherein the inorganic complexing agent and oil are present in a ratio of 1:99 to 1.2:1 by weight.

4. A composition according to any preceding claim, wherein the oil is an animal oil, a vegetable oil or a mineral oil or mixtures thereof.

5. A composition according to claim 4, wherein the oil is a marine oil.

6. A composition according to claim 5, wherein the oil is fish oil, whale oil, seal oil, fish liver oil or an oil containing at least one omega-3 fatty acid or mixtures thereof.

7. A composition according to claim 4, wherein the oil is a vegetable oil selected from castor, linseed, sunflower, soybean, olive, peanut, rapeseed, corn, safflower, cottonseed, coconut, palm, palm kernel and mixtures thereof.

8. A composition according to any preceding claim, wherein the inorganic complexing agent has a particle size of 0.1 micrometres to 300 micrometres.

9. A composition according to any preceding claim, wherein the water is present from 10% to 90% by weight of the total composition.

10. A pharmaceutical, cosmetic, personal care, food (edible), agricultural, lubricant or solvent formulation containing a composition according to any one of claims 1 to 9.

11. A pharmaceutical formulation according to claim 10, which is for topical administration.

12. A process for the preparation of a composition according to any one of claims 1 to 9 which process comprises admixing an oil and an inorganic complexing agent until a homogenous mixture of increased viscosity is obtained and admixing water with the mixture to form a uniform emulsion.

### Claims for the following Contracting States : GR

1. A composition which comprises:-
an oil and an inorganic complexing agent selected from magnesium trisilicate, calcium carbonate, calcium silicate, a co-dried gel of aluminium hydroxide and magnesium carbonate, magnesium hydroxide, magnesium carbonate, aluminium hydroxide, ground limestone or ground oyster shells or mixtures thereof, which is particulate form, is insoluble in the oil and is associated with the oil such that the viscosity of the oil is increased; and
water

which oil/agent association and water are in the form of an emulsion.

2. A composition according to claim 1, wherein the inorganic complexing agent is magnesium trisilicate.

3. A composition according to any one of claims 1 and 2, wherein the inorganic complexing agent and oil are present in a ratio of 1:99 to 1.2:1 by weight.

4. A composition according to any preceding claim, wherein the oil is an animal oil, a vegetable oil or a mineral oil or mixtures thereof.

5. A composition according to claim 4, wherein the oil is a marine oil.

6. A composition according to claim 5, wherein the oil is fish oil, whale oil, seal oil, fish liver oil or an oil containing at least one omega-3 fatty acid or mixtures thereof.

7. A composition according to claim 4, wherein the oil is a vegetable oil selected from castor, linseed, sunflower, soybean, olive, peanut, rapeseed, corn, safflower, cottonseed, coconut, palm, palm kernel and mixtures thereof.

8. A composition according to any preceding claim, wherein the inorganic complexing agent has a particle size of 0.1 micrometres to 300 micrometres.

9. A composition according to any preceding claim, wherein the water is present from 10% to 90% by weight of the total composition.

10. A cosmetic, personal care, food (edible), agricultural, lubricant or solvent formulation containing a composition according to any one of claims 1 to 9.

11. A process for the preparation of a composition according to any one of claims 1 to 9 which process comprises admixing an oil and an inorganic complexing agent until a homogenous mixture of increased viscosity is obtained and admixing water with the mixture to form a uniform emulsion.

12. A process for the preparation of a pharmaceutical formulation which process comprises admixing a composition according to any one of claims 1 to 9 with at least one ingredient selected from colourants, humectants, therapeutic agents, fragrances, lubricants, preservatives, emulsifiers, surfactants and neutralising agents.

**Claims for the following Contracting States : ES**

1. A process for the preparation of a composition which comprises:-

an oil and an inorganic complexing agent selected from magnesium trisilicate, calcium carbonate, calcium silicate, a co-dried gel of aluminium hydroxide and magnesium carbonate, magnesium hydroxide, magnesium carbonate, aluminium hydroxide, ground limestone or ground oyster shells or mixtures thereof, which is in particulate form, is insoluble in the oil and which is associated with the oil such that the viscosity of the oil is increased; and

water

which oil/agent association and water are in the form of an emulsion, which process comprises admixing the oil and the inorganic complexing agent until a homogenous mixture of increased viscosity is obtained and admixing the water with the mixture to form a uniform emulsion.

2. A process according to claim 1, wherein the inorganic complexing agent is magnesium trisilicate.

3. A process according to any one of claims 1 and 2, wherein the inorganic complexing agent and oil are present in a ratio of 1:99 to 1.2:1 by weight.

4. A process according to any preceding claim, wherein the oil is an animal oil, a vegetable oil or a mineral oil or mixtures thereof.

5. A process according to claim 4, wherein the oil is a marine oil.

6. A process according to claim 5, wherein the oil is fish oil, whale oil, seal oil, fish liver oil or an oil containing at least one omega-3 fatty acid or mixtures thereof.

7. A process according to claim 4, wherein the oil is a vegetable oil selected from castor, linseed, sunflower, soybean, olive, peanut, rapeseed, corn, safflower, cottonseed, coconut, palm, palm kernel and mixtures thereof.

8. A process according to any preceding claim, wherein the inorganic complexing agent has a particle size of 0.1 micrometres to 300 micrometres.

9. A process according to any preceding claim, wherein the water is present from 10% to 90% by weight of the total composition.

10. A process for the preparation of a pharmaceutical, cosmetic, personal care, food (edible), agricultural, lubricant or solvent formulation which process comprises admixing a composition prepared according to any one of claims 1 to 9 with at least one ingredient selected from colourants, humectants, therapeutic agents, fragrances, lubricants, preservatives, emulsifiers, surfactants and neutralising agents.

11. A process for the preparation of a pharmaceutical formulation according to claim 10, which pharmaceutical formulation is for topical administration.


## Revendications

**Revendications pour les Etats contractants suivants : AT, BE, FR, DE, IT, LU, NL, SE, CH et GB**

1. Une composition qui comprend:

une huile et un agent complexant minéral choisi parmi: trisilicate de magnésium, carbonate de calcium, silicate de calcium, un gel co-séché d'hydroxyde d'aluminium et de carbonate de magnésium, hydroxyde de magnésium, carbonate de magnésium, hydroxyde d'aluminium, chaux broyée ou coquilles d'huîtres broyées ou leurs mélanges, qui se trouve sous une forme particulaire insoluble dans l'huile et qui est associé à l'huile de façon à augmenter la viscosité de l'huile; et

de l'eau,

cette association huile/agent et eau étant sous la forme d'une émulsion.

2. Une composition selon la revendication 1, caractérisée en ce que l'agent complexant minéral est le trisilicate de magnésium.

3. Une composition selon les revendications 1 et 2, caractérisée en ce que l'agent complexant minéral et l'huile sont présents dans un rapport de 1/99 à 1,2/1 en poids.

4. Une composition selon l'une quelconque des revendications précédentes, caractérisée en ce que l'huile est une huile végétale ou une huile minérale ou des mélanges de celles-ci.

5. Une composition selon la revendication 4, caractérisée en ce que l'huile est une huile marine.

6. Une composition selon la revendication 5, caractérisée en ce que l'huile est: huile de poisson, huile de baleine, huile de phoque, huile de foie de poisson ou une huile contenant au moins un acide gras oméga-3 ou leurs mélanges.

7. Une composition selon la revendication 4, caractérisée en ce que l'huile est une huile végétale choisie parmi: huile de ricin, huile de lin, huile de tournesol, huile de soja, huile d'olive, huile d'arachide, huile de colza, huile de maïs, huile de safran, huile de coton, huile de noix de coco, huile de palme, huile de noyau de palme et leurs mélanges.

8. Une composition selon l'une quelconque des revendications précédentes, caractérisée en ce que les dimensions des particules de l'agent complexant minéral sont comprises entre 0,1 et 300 μm.

9. Une composition selon l'une quelconque des revendications précédentes, caractérisée en ce que l'eau est présente en une quantité de 10% à 90% par rapport au poids de la composition totale.

10. Une formulation pharmaceutique, cosmétique, d'hygiène personnelle, alimentaire (comestible), agricole, lubrifiante ou solvante, contenant une composition selon l'une quelconque des revendications 1 à 9.

11. Une formulation pharmaceutique selon la revendication 10, qui est destinée à une administration topique.

12. Un procédé de préparation d'une composition selon l'une quelconque des revendications 1 à 9, ce procédé consistant à mélanger une huile et un agent complexant minéral jusqu'à ce que l'on obtienne un mélange homogène de viscosité accrue et à mélanger de l'eau avec ce mélange pour former une émulsion uniforme.


**Revendications pour l'Etat contractant suivant : GR**

1. Un procédé de préparation d'une composition qui comprend:

une huile et un agent complexant minéral choisi parmi: trisilicate de magnésium, carbonate de calcium, silicate de calcium, un gel co-séché d'hydroxyde d'aluminium et de carbonate de magnésium, hydroxyde de magnésium, carbonate de magnésium, hydroxyde d'aluminium, chaux broyée ou coquilles d'huîtres broyées ou leurs mélanges, qui se trouve sous une forme particulaire insoluble dans l'huile et qui est associé à l'huile de façon à augmenter la viscosité de l'huile; et

de l'eau,

cette association huile/agent et eau étant sous la forme d'une émulsion, ce procédé consistant à mélanger l'huile et l'agent complexant minéral jusqu'à ce que l'on obtienne un mélange homogène de viscosité accrue et à mélanger l'eau avec ce mélange pour former une émulsion uniforme.

2. Un procédé selon la revendication 1, caractérisé en ce que l'agent complexant minéral est le trisilicate de magnésium.

3. Un procédé selon les revendications 1 et 2, caractérisé en ce que l'agent complexant minéral et l'huile sont présents dans un rapport de 1/99 à 1,2/1 en poids.

4. Un procédé selon l'une quelconque des revendications précédentes, caractérisé en ce que l'huile est une huile végétale ou une huile minérale ou des mélanges de celles-ci.

5. Un procédé selon la revendication 4, caractérisé en ce que l'huile est une huile marine.

6. Un procédé selon la revendication 5, caractérisé en ce que l'huile est: huile de poisson, huile de baleine, huile de phoque, huile de foie de poisson ou une huile contenant au moins un acide gras oméga-3 ou leurs mélanges.

7. Un procédé selon la revendication 4, caractérisé en ce que l'huile est une huile végétale choisie parmi: huile de ricin, huile de lin, huile de tournesol, huile de soja, huile d'olive, huile d'arachide, huile de colza, huile de maïs,

8. Une composition selon l'une quelconque des revendications précédentes, caractérisée en ce que les dimensions des particules de l'agent complexant minéral sont comprises entre 0,1 et 300 μm.

9. Une composition selon l'une quelconque des revendications précédentes, caractérisée en ce que l'eau est présente en une quantité de 10% à 90% par rapport au poids de la composition totale.

10. Une formulation pharmaceutique, cosmétique, d'hygiène personnelle, alimentaire (comestible), agricole, lubrifiante ou solvante, contenant une composition selon l'une quelconque des revendications 1 à 9.

11. Un procédé de préparation d'une composition selon l'une quelconque des revendications 1 à 9, ce procédé consistant à mélanger une huile et un agent complexant minéral jusqu'à ce que l'on obtienne un mélange homogène de viscosité accrue et à mélanger de l'eau avec ce mélange pour former une émulsion uniforme.

12. Un procédé de préparation d'une formulation pharmaceutique, ce procédé consistant à mélanger une composition selon l'une quelconque des revendications 1 à 9 avec au moins un ingrédient choisi parmi des colorants, humectants, agents thérapeutiques, parfums, lubrifiants, conservateurs, émulsifiants, agents tensioactifs et agents de neutralisation.

**Revendications pour l'Etat contractant suivant : ES**

1. Une composition qui comprend:
   une huile et un agent complexant minéral choisi parmi: trisilicate de magnésium, carbonate de calcium, silicate de calcium, un gel co-séché d'hydroxyde d'aluminium et de carbonate de magnésium, hydroxyde de magnésium, carbonate de magnésium, hydroxyde d'aluminium, chaux broyée ou coquilles d'huîtres broyées ou leurs mélanges, qui se trouve sous une forme particulaire insoluble dans l'huile et qui est associé à l'huile de façon à augmenter la viscosité de l'huile; et
   de l'eau,
   cette association huile/agent et eau étant sous la forme d'une émulsion.

2. Une composition selon la revendication 1, caractérisée en ce que l'agent complexant minéral est le trisilicate de magnésium.

3. Une composition selon les revendications 1 et 2, caractérisée en ce que l'agent complexant minéral et l'huile sont présents dans un rapport de 1/99 à 1,2/1 en poids.

4. Une composition selon l'une quelconque des revendications précédentes, caractérisée en ce que l'huile est une huile végétale ou une huile minérale ou des mélanges de celles-ci.

5. Une composition selon la revendication 4, caractérisée en ce que l'huile est une huile marine.

6. Une composition selon la revendication 5, caractérisée en ce que l'huile est: huile de poisson, huile de baleine, huile de phoque, huile de foie de poisson ou une huile contenant au moins un acide gras oméga-3 ou leurs mélanges.

7. Une composition selon la revendication 4, caractérisée en ce que l'huile est une huile végétale choisie parmi: huile de ricin, huile de lin, huile de tournesol, huile de soja, huile d'olive, huile d'arachide, huile de colza, huile de maïs, huile de safran, huile de coton, huile de noix de coco, huile de palme, huile de noyau de palme et leurs mélanges.

8. Un procédé selon l'une quelconque des revendications précédentes, caractérisé en ce que les dimensions des particules de l'agent complexant minéral sont comprises entre 0,1 et 300 μm.

9. Un procédé selon l'une quelconque des revendications précédentes, caractérisé en ce que l'eau est présente en une quantité de 10% à 90% par rapport au poids de la composition totale.

10. Un procédé de préparation d'une formulation pharmaceutique, cosmétique, d'hygiène personnelle, alimentaire (comestible), agricole, lubrifiante ou solvante, ce procédé consistant à mélanger une composition préparée selon l'une quelconque des revendications 1 à 9 avec au moins un ingrédient choisi parmi des colorants, humectants, agents thérapeutiques, parfums, lubrifiants, conservateurs, émulsifiants, agents tensioactifs et agents de neutralisation.

11. Un procédé de préparation d'une formulation pharmaceutique selon la revendication 10, cette formulation pharmaceutique étant destinée à une administration topique.

**Patentansprüche**

**Patentansprüche für folgende Vertragsstaaten : AT, BE, CH, DE, FR, GB, IT, LI, LU, NL und SE**

1. Zusammensetzung welche umfaßt:

ein Öl und einen anorganischen Komplexbildner, ausgewählt aus Magnesiumtrisilikat, Calciumcarbonat, Calciumsilikat, einem gemeinsam getrockneten Gel von Aluminiumhydroxid und Magnesiumcarbonat, Magnesiumhydroxid, Magnesiumcarbonat, Aluminiumhydroxid, gemahlenem Kalkstein oder gemahlenen Austernschalen oder Mischungen davon, welcher Partikelform aufweist, im Öl unlöslich ist und so mit dem Öl verbunden ist, daß die Viskosität des Öls erhöht ist,

und Wasser;

welche Öl/Komplexbildner-Zusammensetzung und Wasser die Form einer Emulsion aufweisen.

2. Zusammensetzung nach Anspruch 1, worin der anorganische Komplexbildner Magnesiumtrisilikat ist.

3. Zusammensetzung nach Anspruch 1 und 2, worin der anorganische Komplexbildner und das Öl in einem Verhältnis von 1:99 bis 1,2:1, bezogen auf das Gewicht, anwesend sind.

4. Zusammensetzung nach irgendeinem vorhergehenden Anspruch, worin das Öl ein tierisches Öl, ein Pflanzenöl oder ein Mineralöl oder Mischungen davon ist.

5. Zusammensetzung nach Anspruch 4, worin das Öl ein Tran ist.

6. Zusammensetzung nach Anspruch 5, worin das Öl Tran, Walfischtran, Robbentran, Lebertran oder ein zumindest eine Omega-3-Fettsäure enthaltendes Öl oder Mischungen davon ist.

7. Zusammensetzung nach Anspruch 4, worin das Öl ein Pflanzenöl ausgewählt aus Rizinus-, Lein-, Sonnenblumen-, Sojabohnen-, Oliven-, Erdnuß-, Raps-, Mais-, Saflor-, Baumwollsamen-, Kokos-, Palm-, Palmkernöl und Mischungen davon ist.

8. Zusammensetzung nach irgendeinem vorhergehenden Anspruch, worin der anorganische Komplexbildner eine Teilchengröße von 0,1 Mikrometern bis 300 Mikrometern aufweist.

9. Zusammensetzung nach irgendeinem vorhergehenden Anspruch, worin das Wasser in einer Menge von 10 bis 90 Gew.-% der Gesamtzusammensetzung anwesend ist.

10. Arzneimittel-, Kosmetik-, Pflege-, Nahrungsmittel-(eßbar), Landwirtschafts-, Gleitmittel- oder Lösungsmittelformulierung, welche eine Zusammensetzung nach irgendeinem der Ansprüche 1 bis 9 umfaßt.

11. Pharmazeutische Formulierung nach Anspruch 10, welche zur topischen Anwendung bestimmt ist.

12. Verfahren zur Herstellung einer Zusammensetzung nach irgendeinem der Ansprüche 1 bis 9, welches Verfahren das Mischen eines Öls und eines anorganischen Komplexbildners bis zum Erhalt einer homogenen Mischung mit erhöhter Viskosität und das Mischen von Wasser mit der Mischung zur Bildung einer gleichförmigen Emulsion umfaßt.

**Patentansprüche für folgenden Vertragsstaat : GR**

1. Zusammensetzung welche umfaßt:

ein Öl und einen anorganischen Komplexbildner, ausgewählt aus Magnesiumtrisilikat, Calciumcarbonat, Calciumsilikat, einem gemeinsam getrockneten Gel von Aluminiumhydroxid und Magnesiumcarbonat, Magnesiumhydroxid, Magnesiumcarbonat, Aluminiumhydroxid, gemahlenem Kalkstein oder gemahlenen Austernschalen oder Mischungen davon, welcher Partikelform aufweist, im Öl unlöslich ist und so mit dem Öl verbunden ist, daß die Viskosität des Öls erhöht ist, und

wasser;

welche Öl/Komplexbildner-Zusammensetzung und Wasser die Form einer Emulsion aufweisen.

2. Zusammensetzung nach Anspruch 1, worin der anorganische Komplexbildner Magnesiumtrisilikat ist.

3. Zusammensetzung nach irgendeinem der Ansprüche 1 und 2, worin der anorganische Komplexbildner und das Öl in einem Verhältnis von 1:99 bis 1,2:1, bezogen auf das Gewicht, anwesend sind.

4. Zusammensetzung nach irgendeinem vorhergehenden Anspruch, worin das Öl ein tierisches Öl, ein Pflanzenöl oder ein Mineralöl oder Mischungen davon ist.

5. Zusammensetzung nach Anspruch 4, worin das Öl ein Tran ist.

6. Zusammensetzung nach Anspruch 5, worin das Öl Tran, Walfischtran, Robbentran, Lebertran oder ein zumindest eine Omega-3-Fettsäure enthaltendes Öl oder Mischungen davon ist.

7. Zusammensetzung nach Anspruch 4, worin das Öl ein Pflanzenöl ausgewählt aus Rizinus-, Lein-, Sonnenblumen-, Sojabohnen-, Oliven-, Erdnuß-, Raps-, Mais-, Saflor-, Baumwollsamen-, Kokos-, Palm-, Palmkernöl und Mischungen davon ist.

8. Zusammensetzung nach irgendeinem vorhergehenden Anspruch, worin der anorganische Komplexbildner eine Teilchengröße von 0,1 Mikrometern bis 300 Mikrometern aufweist.

9. Zusammensetzung nach irgendeinem vorhergehenden Anspruch, worin das Wasser in einer Menge von 10 bis 90 Gew.-% der Gesamtzusammensetzung anwesend ist.

10. Kosmetik-, Pflege-, Nahrungsmittel-(eßbar), Landwirtschafts-, Gleitmittel- oder Lösungsmittelformulierung, welche eine Zusammensetzung nach irgendeinem der Ansprüche 1 bis 9 umfaßt.

11. Verfahren zur Herstellung einer Zusammensetzung nach irgendeinem der Ansprüche 1 bis 9, welches Verfahren das Mischen eines Öls und eines anorganischen Komplexbildners bis zum Erhalt einer homogenen Mischung mit erhöhter Viskosität und das Mischen von Wasser mit der Mischung zur Bildung einer gleichförmigen Emulsion umfaßt.

12. Verfahren zur Herstellung einer pharmazeutischen Formulierung, welches Verfahren das Mischen einer Zusammensetzung nach irgendeinem der Ansprüche 1 bis 9 mit zumindest einem Ingrediens ausgewählt aus Farbstoffen, Feuchthaltemitteln, Therapeutika, Duftstoffen, Gleitmitteln, Konservierungsmitteln, Emulgatoren, grenzflächenaktiven Mitteln und Neutralisierungsmitteln umfaßt.

**Patentansprüche für folgenden Vertragsstaat : ES**

1. Verfahren zur Herstellung einer Zusammensetzung, welche umfaßt:

ein Öl und einen anorganischen Komplexbildner, ausgewählt aus Magnesiumtrisilikat, Calciumcarbonat, Calciumsilikat, einem gemeinsam getrockneten Gel von Aluminiumhydroxid und Magnesiumcarbonat, Magnesiumhydroxid, Magnesiumcarbonat, Aluminiumhydroxid, gemahlenem Kalkstein oder gemahlenen Austernschalen oder Mischungen davon, welcher Partikelform aufweist, im Öl unlöslich ist und so mit dem Öl verbunden ist, daß die Viskosität des Öls erhöht ist, und

Wasser;

welche Öl/Komplexbildner-Zusammensetzung und Wasser die Form einer Emulsion aufweisen, welches Verfahren das Mischen des Öls und des anorganischen Komplexbildners bis zum Erhalt einer homogenen Mischung mit erhöhter Viskosität und das Mischen des Wassers mit der Mischung zur Bildung einer gleichförmigen Emulsion umfaßt.

2. Verfahren nach Anspruch 1, worin der anorganische Komplexbildner Magnesiumtrisilikat ist.

3. Verfahren nach irgendeinem der Ansprüche 1 und 2, worin der anorganische Komplexbildner und das Öl in einem Verhältnis von 1:99 bis 1,2:1, bezogen auf das Gewicht, anwesend sind.

4. Verfahren nach irgendeinem vorhergehenden Anspruch, worin das Öl ein tierisches Öl, ein Pflanzenöl oder ein Mineralöl oder Mischungen davon ist.

5. Verfahren nach Anspruch 4, worin das Öl ein Tran ist.

6. Verfahren nach Anspruch 5, worin das Öl Tran, Walfischtran, Robbentran, Lebertran oder ein zumindest eine Omega-3-Fettsäure enthaltendes Öl oder Mischungen davon ist.

7. Verfahren nach Anspruch 4, worin das Öl ein Pflanzenöl ausgewählt aus Rizinus-, Lein-, Sonnenblumen-, Sojabohnen-, Oliven-, Erdnuß-, Raps-, Mais-, Saflor-, Baumwollsamen-, Kokos-, Palm-, Palmkernöl und Mischungen davon ist.

8. Verfahren nach irgendeinem vorhergehenden Anspruch, worin der anorganische Komplexbildner eine Teilchengröße von 0,1 Mikrometern bis 300 Mikrometern aufweist.

9. Verfahren nach irgendeinem vorhergehenden Anspruch, worin das Wasser in einer Menge von 10 bis 90 Gew.-% der Gesamtzusammensetzung anwesend ist.

10. Verfahren zur Herstellung einer Arzneimittel-, Kosmetik-, Pflege-, Nahrungsmittel- (eßbar), Landwirtschafts-, Gleitmittel- oder Lösungsmittelformulierung, welches Verfahren das Mischen einer nach irgendeinem der Ansprüche 1 bis 9 hergestellten Zusammensetzung mit zumindest einem Ingrediens ausgewählt aus Farbstoffen, Feuchthaltemitteln, Therapeutika, Duftstoffen, Gleitmitteln, Konservierungsmitteln, Emulgatoren, grenzflächenaktiven Mitteln und Neutralisierungsmitteln umfaßt.

11. Verfahren zur Herstellung einer pharmazeutischen Formulierung nach Anspruch 10, welche pharmazeutische Formulierung zur topischen Anwendung bestimmt ist.